(19) Europäisches Patentamt
European Patent Office
Office européen des brevets

(11) Veröffentlichungsnummer : **0 290 903 B1**

# (12) EUROPÄISCHE PATENTSCHRIFT

(45) Veröffentlichungstag der Patentschrift : **11.09.91 Patentblatt 91/37**

(51) Int. Cl.$^5$ : **C07C 49/255**, C07C 45/63, C07C 45/65, C07C 49/175

(21) Anmeldenummer : **88107010.6**

(22) Anmeldetag : **02.05.88**

## (54) Beta-Fluoracyl-beta-halogenvinylalkylether.

(30) Priorität : **12.05.87 DE 3715704**

(43) Veröffentlichungstag der Anmeldung : **17.11.88 Patentblatt 88/46**

(45) Bekanntmachung des Hinweises auf die Patenterteilung : **11.09.91 Patentblatt 91/37**

(84) Benannte Vertragsstaaten : **BE CH DE FR GB IT LI NL**

(56) Entgegenhaltungen :
**EP-A- 0 091 022**
**EP-A- 0 161 841**

(56) Entgegenhaltungen :
**US-A- 4 076 749**
**JOURNAL OF SYNTHETIC ORGANIC CHEMISTRY, Nr. 4, April 1988, Seiten 274-277, Georg Thieme Verlag; L.-F. TIETZE et al.: "Highly efficient syntheses of Alkyl 3,3-Dialkoxypropanotes, Alkyl 4-Ethoxy-2-oxo-3-butenoates, and monoprotected malonaldehydes"Seite 276**

(73) Patentinhaber : **BAYER AG**
**W-5090 Leverkusen 1 Bayerwerk (DE)**

(72) Erfinder : **Heine, Hans-Georg, Dr.**
**Am Heckerhof 14**
**W-4150 Krefeld (DE)**
Erfinder : **Ooms, Pieter, Dr.**
**Doerperhofstrasse 16**
**W-4150 Krefeld (DE)**

**EP 0 290 903 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue β-Fluoracyl-β-halogenvinylalkylether und Verfahren zu ihrer Herstellung. Die neuen β-Fluoracyl-β-halogenvinylalkylether können beispielsweise als Zwischenprodukte für die Herstellung biologisch wirksamer Verbindungen verwendet werden.

Es ist bekannt, Ethylvinylether zu halogenieren und das Halogenierungsprodukt unter Bildung von β-Halogenvinylethylether zu dehydrohalogenieren [Angew. Chem. Int. Ed. 8, 295 (1969) ; Org. Prep. Proc. Int. 17, 410 (1985) ; J. Org. Chem. 51, 3577 (1986)]. Die Herstellung von β-Acyl-β-halogenvinylethylethern auf diesem Weg ist nicht bekannt. Bekannt ist weiterhin die Synthese des 3-Chlor-4-methoxy-pent-3-en-2-ons durch Chlorierung des Pentan-2.4-dions und folgende Umsetzung des 3-Chlorpentan-2.4-dions mit Dimethylsulfat/Kaliumcarbonat in Aceton [Bull. Soc. Chim. Belg. 87, 143 (1978)]. Ähnlich ist 2-Chlor-3-methoxy-1-phenylbut-2-en-1-on im Gemisch mit seinen drei Strukturisomeren erhalten worden. Bekannt ist weiterhin die Herstellung von 3-Chlor-4.4-diethoxybutan-2-on aus Chloraceton [J. Org. Chem. 46, 2557/1981)].

Die β-Fluoracyl-β-halogenvinyl-alkylether der Formel (I) sind bislang noch nicht hergestellt worden.

Es wurden neue β-Fluoracyl-β-halogenvinylalkylether der allgemeinen Formel I

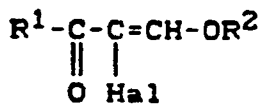 (I)

in welcher

$R^1$ für einen fluorierten Alkylrest mit 1 bis 9 Kohlenstoffatomen steht,
Hal für ein Halogenatom steht und
$R^2$ für einen Alkylrest mit 1 bis 7 Kohlenstoffatomen steht,

gefunden.

Weiterhin wurde gefunden, daß man die neuen β-Fluoracyl-β-halogenvinylalkylether der allgemeinen Formel (I)

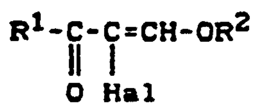 (I)

in welcher

$R^1$ für einen fluorierten Alkylrest mit 1 bis 9 Kohlenstoffatomen steht,
Hal für ein Halogenatom steht und
$R^2$ für einen Alkylrest mit 1 bis 7 Kohlenstoffatomen steht,

erhält,
indem man einen β-Fluoracylvinylalkylether der Formel (II)

$$R^1\text{-CO-CH=CH-OR}^2 \quad [II]$$

worin

$R^1$ und $R^2$ die oben angegebene Bedeutung haben,
mit einem Halogenierungsmittel bei Temperaturen von −70°C bis +80°C gegebenenfalls in Gegenwart eines Verdünnungsmittels umsetzt, das resultierende Halogenierungsprodukt der allgemeinen Formel (III)

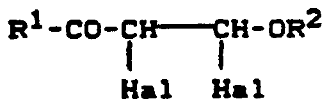

(III)

gegebenenfalls isoliert
und bei einer Temperatur von −20°C bis +100°C dehydrohalogeniert.

Die neuen β-Fluoracyl-β-halogenvinylalkylether sind durch die Formel (I) allgemein definiert.

Hierbei stehen vorzugsweise

$R^1$ für einen fluorierten Alkylrest mit 1 bis 3 Kohlenstoffatomen
Hal für Chlor oder Brom und
$R^2$ für einen Alkylrest mit 1 bis 4 Kohlenstoffatomen.

Im einzelnen seien außer den bei den Herstellungsbeispielen genannten Verbindungen die folgenden β-Fluoracyl-β-halogenvinylalkylether der allgemeinen Formel (I) genannt:

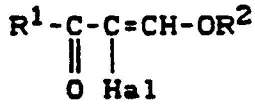

(I)

| $R^1$ | Hal | $OR^2$ |
|---|---|---|
| $CF_3$ | F | $OCH_3$ |
| $CF_3$ | F | $OC_2H_5$ |
| $CF_3$ | F | $OC_4H_9$(n) |
| $CF_3$ | Cl | $OC_3H_7$(i) |
| $CF_3$ | Cl | $OC_3H_7$(n) |
| $C_2F_5$ | Cl | $OC_2H_5$ |
| $C_2F_5$ | Cl | $OCH_3$ |
| $C_2F_5$ | Cl | $OC_4H_9$(n) |
| $C_2F_5$ | F | $OC_2H_5$ |
| $CHF_2$ | Cl | $OC_2H_5$ |
| $CHF_2$ | Br | $OC_2H_5$ |
| $CClF_2$ | Cl | $OC_2H_5$ |
| $CFCl_2$ | Cl | $OC_2H_5$ |
| $CClF_2$ | Br | $OC_2H_5$ |
| $CFCl_2$ | Br | $OC_2H_5$ |
| $C_2F_5$ | Br | $OC_2H_5$ |
| $C_2Cl_2F_3$ | Cl | $OC_2H_5$ |
| $C_3F_7$ | Cl | $OC_2H_5$ |
| $C_3F_7$ | Br | $OC_2H_5$ |

Die Dehydrohalogenierung der gegebenenfalls isolierten Verbindungen der Formel (III) zu den erfindungsgemäßen Verbindungen der Formel (I) kann in Abhängigkeit vom Halogenierungsmittel entweder durch Zusatz

einer organischen Base (Hal = Halogenatom allgemein) oder durch thermische Dehydrohalogenierung (Hal = Chlor) durchgeführt werden.

Im Falle der Dehydrohalogenierung durch Basenzusatz sowie Einsatz von 4-Ethoxy-1.1.1-trifluor-but-3-en-2-on als Komponente (II, elementarem Brom als Halogenierungsmittel und Triethylamin als Dehydrohalogenierungsmittel läßt sich die Reaktionssequenz wie folgt darstellen :

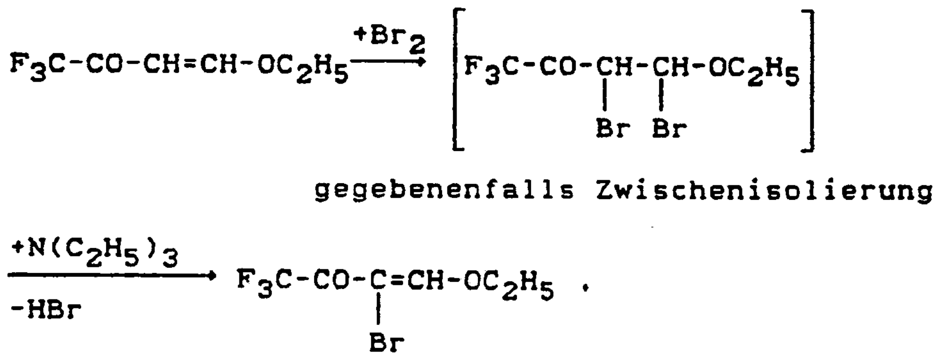


Im Falle der thermischen Dehydrohalogenierung sowie Einsatz von 4-Ethoxy-1.1.1-trifluorbut-3-en-2-on als Komponente (II) und Sulfurylchlorid als Halogenierungsmittel läßt sich die Reaktionssequenz wie folgt darstellen :

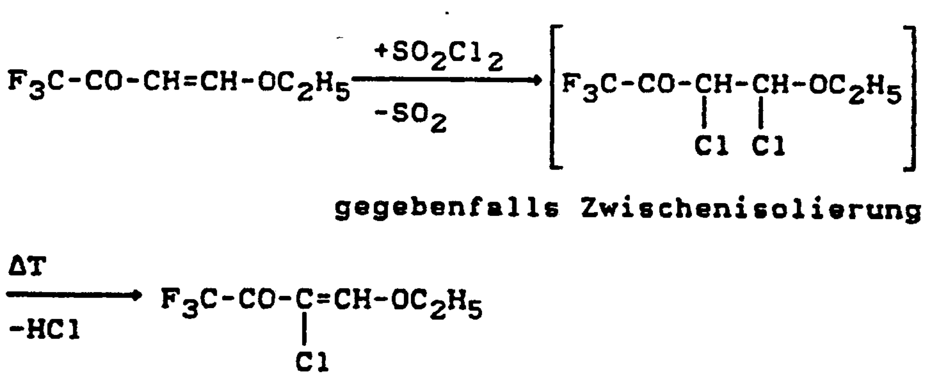


Die für die Herstellung der neuen β-Fluoracyl-β-halogenvinylalkylether der allgemeinen Formel I benötigten Ausgangsverbindungen der allgemeinen Formel II sind bekannt (Chem. Lett. 1976, 499) oder lassen sich in Anlehnung an beschriebene analoge Umsetzungen (Synthesis 1986, 69, 340) synthetisieren.

Als Beispiele seien genannt :

4-Ethoxy-1.1.1-trifluorbut-3-en-2-on
4-n-Butoxy-1.1.1-trifluorbut-3-en-2-on
5-Ethoxy-1.1.1.2.2-pentafluorpent-4-en-3-on
4-Methoxy-1.1.1-trifluorbut-3-en-2-on
6-Ethoxy-1.1.1.2.2.3.3-heptafluorhex-5-en-4-on
13-Ethoxy-1.1.1.2.2.3.3.4.4.5.5.6.6.7.7.8.8.9.9.-nonadecafluortridec-12-en-11-on
4-Ethoxy-1.1-difluorbut-3-en-2-on
4-Ethoxy-1-chlor-1.1-difluorbut-3-en-2-on
4-Ethoxy-1.1-dichlor-1-fluorbut-3-en-2-on
5-Ethoxy-2.2-dichlor-1.1.1-trifluorpent-4-en-3-on.

Zur Halogenierung der Verbindungen der Formel (II) können übliche Halogenierungsmittel eingesetzt werden, wie z.B. Chlor, Brom, Pyridiniumperbromid, N-Bromsuccinimid, N-Chlorsuccinimid oder Sulfurylchlorid.

Die Dehydrohalogenierung kann allgemein durch organische Basen erfolgen. Solche Basen sind beispielsweise Alkalialkoholate, wie Natriummethylat, Natriumethylat, Kalium-tert.-butylat, Natriumisopropylat oder tert. Amine, wie Triethylamin, Pyridin, Dimethylanilin, Methyl-dicyclohexylamin, Triethylendiamin (DABCO) oder Amidine, wie 1.5-Diazabicyclo[5.4.0]-undec-5-en (DBU), 1.4-Diazabicyclo[4.3.0]-non-4-en (DBN). Besonders bevorzugt wird Triethylamin eingesetzt.

Bei der Einwirkung der Base erfolgt unter den Reaktionsbedingungen keine weitere Eliminierung von Halogenwasserstoff zu 1-Fluoracylethinylalkylethern und keine Substitution des vinylständigen Halogenrestes.

Überraschenderweise kann die Dehydrohalogenierung auch thermisch durchgeführt werden, insbesondere dann, wenn dehydrochloriert wird. So spaltet z.B. das aus 4-Ethoxy-1.1.1-trifluorbut-3-en-2-on und beispielsweise Sulfurylchlorid erhaltene Chlorierungsrohprodukt der allgemeinen Formel III ($R^1$=$CF_3$, Hal=Cl, $R^2$=$C_2H_5$) beim Versuch der Destillation bei 133 mbar unerwartet vollständig Chlorwasserstoff ab unter Bildung des 3-Chlor-4-ethoxy-1.1.1-trifluorbut-3-en-2-ons. Ein analoges Verhalten zeigt das entsprechende Bromierungsrohprodukt nicht.

Als Verdünnungsmittel sowohl für die Halogenierung als auch für die Dehydrohalogenierung durch organische Basen sind inerte, aprotische, organische Lösungsmittel geeignet. Hierzu zählen z.B. Kohlenwasserstoffe wie Toluol, Pentan, n-Hexan, Petrolether, Dichlormethan oder Ether wie Diethylether Dioxan, Methyl-tert.-butylether, 1.2-Dimethoxyethan oder Ester wie Essigsäuremethylester, Essigsäureethylester, Dimethylcarbonat. Bei der thermisch durchgeführten Dehydrohalogenierung wird vorzugsweise ohne Verdünnungsmittel gearbeitet.

Die Reaktionstemperatur für die Halogenierung liegt in einem Bereich von etwa −70°C bis +80°C, bevorzugt von etwa −20°C bis +20°C. Für viele Halogenierungen genügen Temperaturen von 0°C bis +20°C. Die Halogenierung wird vorzugsweise unter Normaldruck durchgeführt. Analoge Bedingungen gelten auch für die Reaktionstemperatur der Dehydohalogenierung mit organischen Basen, insbesondere mit tert. Aminen und Amidinen. Wird thermisch dehydrohalogeniert, so liegt die Reaktionstemperatur höher. Im allgemeinen wird dabei im Temperaturbereich von +70°C bis +100°C dehydrohalogeniert. Die Reaktionszeit liegt normalerweise zwischen 0.5 und 5 Stunden. Sie kann aber auch bis zu 24 Stunden betragen, wenn z.B. relativ reaktionsträge tert. Amine als Basen für die Dehydrohalogenierung verwendet werden.

Zur Halogenierung werden im allgemeinen pro Mol Verbindung (II) ein Mol Halogenierungsmittel verwendet. Man kann aber auch einen geringen Überschuß an Halogenierungsmittel, insbesondere bei der Chlorierung mit Sulfurylchlorid, anwenden. Wird zur Dehydrohalogenierung eine organische Base verwendet, so beträgt das Molverhältnis von Base zur Verbindung der allgemeinen Formel III mindestens 1 : 1 und liegt vorzugsweise bei 1.1 : 1 und höchstens bei 1.5 : 1.

Das erfindungsgemäße Verfahren wird im allgemeinen in der Weise durchgeführt, daß man eine Verbindung der allgemeinen Formel II gegebenenfalls in einem Verdünnungsmittel vorlegt und unter Rühren das Halogenierungsmittel gegebenenfalls in einem Verdünnungsmittel zutropft. Im allgemeinen tritt eine exothere Wärmetönung auf. Die Geschwindigkeit der Zugabe des Halogenierungsmittels richtet sich nach seinem Umsatz. d.h. erst wenn zuvor eingetragenes Halogenierungsmittel umgesetzt ist, erfolgt die Zugabe einer weiteren Menge Halogenierungsmittel. Nach beendeter Zugabe rührt man im allgemeinen nach und nimmt dann die Dehydrohalogenierung der Verbindung der allgemeinen Formel III gegebenenfalls nach Zwischenisolierung vor.

Die Dehydrohalogenierung durch organische Basen wird in der Weise durchgeführt, daß man zu dem im allgemeinen nicht isolierten rohen Halogenierungsprodukt gegebenenfalls in einem Verdünnungsmittel vorzugsweise bei Temperaturen von −20°C bis +20°C die organische Base unter Rühren zutropft. Man kann die Reaktanden aber auch in umgekehrter Reihenfolge zusammengeben, d.h. man legt die organische Base gegebenenfalls in einem Verdünnungsmittel vor und tropft das Halogenierungsprodukt der allgemeinen Formel III in einem Verdünnungsmittel unter Rühren zu. Im allgemeinen ist die Dehydrohalogenierung nach Zugabe der erforderlichen Menge organischer Base beendet, so daß sich ein mehrstündiges Nachrühren des Reaktionsgemisches erübrigt. Das Aufarbeiten erfolgt in üblicher Weise : Die Reaktionslösung wird durch Zugabe von Säure, beispielsweise von Salzsäure, neutralisiert oder schwach sauer gestellt, die organische Phase durch Zugabe eines üblichen organischen Lösungsmittels verdünnt, gewaschen, getrocknet und eingedampft. Durch fraktionierende Destillation unter vermindertem Druck wird die neue Verbindung der allgemeinen Formel I isoliert.

Für den Fall der thermischen Abspaltung von Chlorwasserstoff wird im allgemeinen so verfahren, daß das Rohprodukt der Chlorierung unter vermindertem Druck erhitzt wird, bis die Chlorwasserstoffabspaltung beendet ist. Im allgemeinen erhitzt man das Rohprodukt der Chlorierung im Wasserstrahlvakuum zum Rückfluß und destilliert anschließend fraktionierend. Oftmals erübrigt sich das Erhitzen unter Rückfluß und die Dehydrochlorierung erfolgt bereits bei der Destillation unter vermindertem Druck. Durch Redestillation wird ein einheitliches Produkt der allgemeinen Formel I (Hal=Cl) erhalten.

Die nach dem erfindungsgemäßen Verfahren erhaltenen β-Fluoracyl-β-halogenvinylalkylether der allgemeinen Formel I sind Flüssigkeiten oder niedrig schmelzende Verbindungen. Aufgrund der vielfältigen Funktionalität (Enolether, verkappte α-Halogenketone, verkappte 1.3-Dicarbonylverbindungen mit unterschiedlich reaktiven Carbonylgruppen, vinyloge Carbonsäureester) sind die erfindungsgemäßen Verbindungen der allgemeinen Formel I wichtige Ausgangsprodukte für die Herstellung biologisch wirksamer Verbindungen.

Als Beispiel für die Weiterverarbeitung der erfindungsgemäßen Verbindungen sei aufgeführt :

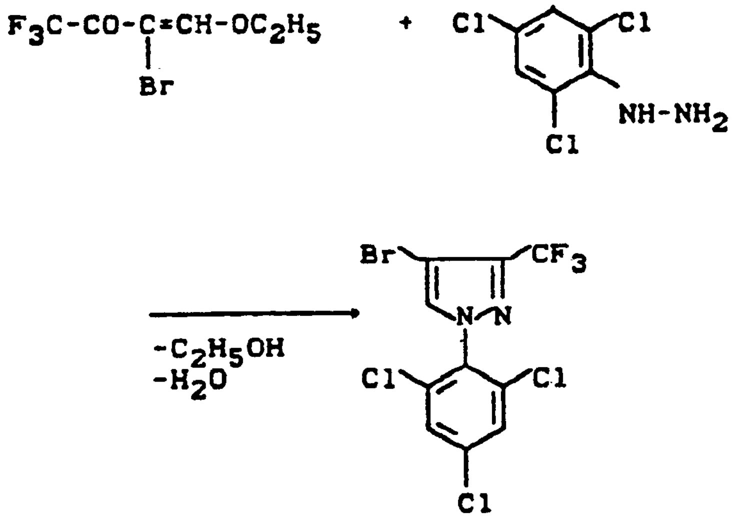


Herstellungsbeispiele

Beispiel 1

Zu der Lösung von 25.5 g (0.15 Mol) 4-Ethoxy-1.1.1-trifluorbut-3-en-2-on in 150 ml Dichlormethan werden bei 0°C 21.0 g (0.165 Mol) Sulfurylchlorid in 90 ml Dichlormethan unter Rühren innerhalb von 30 Min getropft. Man rührt 3 Stunden bei 0°C nach, destilliert das Lösungsmittel und nichtumgesetztes Sulfurylchlorid im Wasserstrahlvakuum weitgehend ab und erhält 38.3 g hellgelbes Öl. Die Konstitution folgt aus dem $^1$H-NMR-Spektrum ($CDCl_3$), das im Einklang mit der Struktur eines etwa 1 : 1-Gemisches der diastereomeren 3.4-Dichlor-4-ethoxy-1.1.1-trifluorbutan-2-one Signale zwischen 1.1-1.5, 3.5-4.2, 4.75-5.0 und 5.75-5.9 ppm im Verhältnis 3 : 2 : 1 : 1 zeigt. Der Anteil an 3-Chlor-4-ethoxy-1.1.1-trifluorbu-3-en-2-on im Gemisch beträgt weniger als 5%. Durch Destillieren bei 1,3 mbar läßt sich das Diastereomerengemisch unzersetzt von Lösungsmittelresten befreien.

Beispiel 2

Das nach Beispiel 1 erhaltene 3.4-Dichlor-4-ethoxy-1.1.1-trifluorbutan-2-on (38.0 g) wird langsam im Wasserstrahlvakuum destilliert und liefert 23.9 g schwach gelb gefärbtes 3-Chlor-4-ethoxy-1.1.1-trifluorbut-3-en-2-on vom Siedepunkt 84-87°C/13,3 mbar $n_D^{20}$ = 1.4380.
$^1$H-NMR ($CDCl_3$) : δ = 1.55 t (3H, J = 8 Hz), 4.40 q (2H) und 8.00 ppm s (1H)
IR ($CCl_4$) : $V_{CO}$ = 1710 $cm^{-1}$.

Beispiel 3

Zu 39.0 g (0.2 Mol) 4-n-Butoxy-1.1.1-trifluor-but-3-en-2-on in 200 ml Dichlormethan werden bei 0°C unter Rühren 28.0 g (0.22 Mol) Sulfurylchlorid in 60 ml Dichlormethan unter Rühren getropft. Man rührt 4 Stdn. bei 0°C nach, destilliert Lösungsmittel und nichtumgesetztes Sulfurylchlorid ab und destilliert den Rückstand langsam unter vermindertem Druck. Man erhält 42.5 g schwach gelbgefärbtes 4-n-Butoxy-3-chlor-1.1.1-trifluorbut-3-en-2-on vom Siedepunkt 105-106° C/12 mbar, $n_D^{20}$ = 1.4484.
$^1$H-NMR ($CDCl_3$) : δ = 1.01 t (3H, J = 8 Hz), 1.25-2.05 m (4H), 4.25-4.50 m (2H) und 8.00 ppm s (1H)
IR ($CCl_4$) : $v_{CO}$ = 1710 $cm^{-1}$.
Analog erhält man
3-Chlor-4-methoxy-1.1.1-trifluorbut-3-en-2-on
4-Chlor-5-ethoxy-1.1.1.2.2-pentafluorpent-4-en-3-on
3-Chlor-4-n-propoxy-1.1.1-trifluorbut-3-en-2-on.

Beispiel 4

Zu 8.35 g (0.05 Mol) 4-Ethoxy-1.1.1-trifluor-but-3-en-2-on in 50 ml Pentan werden bei −20°C unter Rühren 8.0 g (0.05 Mol) Brom in 50 ml Pentan getropft. Man rührt 1 Std. bei −20°C nach und tropft anschließend die Lösung von 6.0 g (0.06 Mol) Triethylamin in 150 ml Pentan zu. Nach Rühren über Nacht bei 20°C wird der Reaktionsansatz auf Eis gegeben. Man säuert mit 1n Salzsäure an (pH-Wert 4-5) und extrahiert mit Petrolether. Waschen der organischen Phase mit gesättigter Kochsalzlösung, Trocknen über wasserfreiem Natriumsulfat und Eindampfen der Lösung liefern 12.1 g gelbliche Flüssigkeit, die fraktionierend unter vermindertem Druck destilliert wird. 10.4 g 3-Brom-4-ethoxy-1.1.1-trifluorbut-3-en-2-on vom Siedepunkt 100-102°C/21.3 mbar, $n_D^{20}$ = 1.4680 werden erhalten, die bei −30°C kristallieren.

$^1$H-NMR ($CDCl_3$) : $\delta$ = 1.51 t (3H, J = 8 Hz), 4.45 q (2H) und 8.06 ppm s (1H)

IR ($CCl_4$) : $\nu_{CO}$ = 1710 $cm^{-1}$.

Beispiel 5

In die auf −20°C gekühlte Lösung von 9.75 g (0.05 Mol) 4-n-Butoxy-1.1.1-trifluor-but-3-en-2-on in 50 ml Pentan werden 8.0 g (0.05 Mol) Brom, in 100 ml Pentan gelöst, getropft. Man rührt 1 Std. bei −20°C nach und tropft darauf bei derselben Temperatur die Lösung von 6.0 g (0.06 Mol) Triethylamin in 100 ml Pentan zu. Nach Stehenlassen über Nacht bei 25° gießt man den Ansatz in Eiswasser, säuert mit 1n Salzsäure an und trennt die Phasen. Übliches Aufarbeiten liefert 12.1 g fast einheitliches rohes 3-Brom-4-n-butoxy-1.1.1-trifluorbut-3-en-2-on, das durch Destillieren unter vermindertem Druck von geringen Verunreinigungen befreit wird. Siedepunkt 113-115° C/16 mbar, $n_D^{20}$ = 1,4640.

$^1$H-NMR ($CDCl_3$) : $\delta$ = 1.00 t (3H), 1.25-2.00 m (4H),4.25-4.40 m (2H) und 8.00 ppm s (1H)

IR ($CCl_4$) : $\nu_{CO}$ = 1710 $cm^{-1}$.

Beispiel 6

5.1 g (0.03 Mol) 4-Ethoxy-1.1.1-trifluorbut-3-en-2-on werden in 30 ml n-Hexan mit 5.3 g (0.033 Mol) Brom in 18 ml n-Hexan bei −20°C unter Rühren umgesetzt. Man rührt 1 Std. bei −20°C nach, gießt den Ansatz auf Eis und extrahiert mit Petrolether. Trocknen der organischen Phase über wasserfreiem Natriumsulfat und Eindampfen unter vermindertem Druck liefern 9.6 g rohes 3.4-Dibrom-4-ethoxy-1.1.1-trifluorbutan-2-on (nach $^1$H-NMR-($CDCl_3$)-Analyse hauptsächlich ein Diastereomer). Destillieren unter vermindertem Druck ergibt das lösungsmittelfreie Dibrom-Addukt vom Siedepunkt 43-47° C/16 mbar (Gehalt an 3-Brom-4-ethoxy-1.1.1-trifluorbut-3-en-2-on kleiner als 5%).

Umsetzung des 3.4-Dibrom-4-ethoxy-1.1.1-trifluorbutan-2-ons mit Triethylamin in Pentan gemäß Beispiel 4 ergibt zu 90% 3-Brom-4-ethoxy-1.1.1-trifluorbut-3-en-2-on.

Beispiel 7

Zu der Lösung von 8.4 g (0.05 Mol) 4-Ethoxy-1.1.1-trifluor-but-3-en-2-on in 50 ml Tetrachlormethan gibt man 9.8 g (0.055 Mol) N-Bromsuccinimid und 100 mg Azoisobutyronitril. Anschließend erhitzt man 8 h zum Rückfluß, filtriert die abgekühlte Lösung und engt das Filtrat ein. Man erhält 13.4 g Rohprodukt, dessen Destillation unter vermindertem Druck 9.7 g 3-Brom-4-ethoxy-1.1.1-trifluorbut-3-en-2-on ergibt.

Analog wird bei Verwendung von N-Chlor-succinimid 3-Chlor-4-ethoxy-1.1.1-trifluor-but-3-en-2-on erhalten.

Beispiel 8 : Weiterverarbeitung

2.3 g (0.01 Mol) 3-Brom-4-ethoxy-1.1.1-trifluorbut-3-en-2-on werden zur Lösung von 4.2 g (0.02 Mol) 2.4.6-Trichlorphenylhydrazin in 100 ml Ethanol, die 0.01 Mol Bromwasserstoff enthält, gegeben. Das Gemisch erhitzt man 5 h zum Rückfluß, engt anschließend unter vermindertem Druck ein und versetzt den Rückstand mit Eiswasser. Extrahiert mit Dichlormethan, Neutralwaschen mit gesättigter Kochsalzlösung, Klären über wasserfreiem Natriumsulfat und Einengen liefern 4.6 g Rohproduct, das an 120 g Kieselgel mit Toluol/Petrolether (Verhältnis 1 : 1) chromatographiert wird. 1.2 g 4-Brom-1-(2.4.6-trichlorphenyl)-3-trifluormethylpyrazol vom Schmelzpunkt 93-94° C (aus Petrolether) werden erhalten.

$^1$H-NMR ($CDCl_3$) : $\delta$ = 7.50 s (2H) und 7.64 ppm s (1H).

## Patentansprüche

1. β-Fluoracyl-β-halogenvinylalkylether der allgemeinen Formel (I)

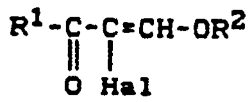

(I)

in welcher

$R^1$ für einen fluorierten Alkylrest mit 1 bis 9 Kohlenstoffatomen steht,
Hal für ein Halogenatom steht und
$R^2$ für einen Alkylrest mit 1 bis 7 Kohlenstoffatomen steht.

2. β-Fluoracyl-β-halogenvinylalkylether gemäß Formel (1) in Anspruch 1), in welcher

$R^1$ für einen fluorierten Alkylrest mit 1 bis 3 Kohlenstoffatomen
Hal für Chlor oder Brom und
$R^2$ für einen Alkylrest mit 1 bis 4 Kohlenstoffatomen steht.

3. β-Fluoracyl-β-halogenvinylether gemäß Anspruch 2), dadurch gekennzeichnet, daß

$R^1$ für einen perfluorierten Alkylrest mit 1 bis 3 Kohlenstoffatomen steht.

4. Verfahren zur Herstellung von β-Fluoracyl-β-halogenvinylalkylether der allgemeinen Formel (I)

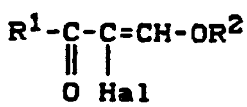

(I)

in welcher

$R^1$ für einen fluorierten Alkylrest mit 1 bis 9 Kohlenstoffatomen steht,
Hal für ein Halogenatom steht und
$R^2$ für einen Alkylrest mit 1 bis 7 Kohlenstoffatomen steht, dadurch gekennzeichnet, daß man β-Fluoracylvinylalkylether der Formel (II)

$$R^1\text{-CO-CH=CH-OR}^2 \quad [II]$$

worin

$R^1$ und $R^2$ die oben angegebene Bedeutung haben, mit einem Halogenierungsmittel bei Temperaturen von –70°C bis +80C gegebenenfalls in Gegenwart eines Verdünnungsmittels umsetzt, das resultierende Halogenierungsprodukt der allgemeinen Formel (III)

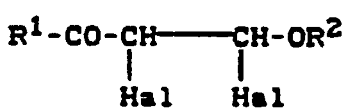

(III)

gegebenenfalls isoliert und bei einer Temperatur von –20°C bis +100°C dehydrohalogeniert.

5. Verfahren gemäß Anspruch 4, dadurch gekennzeichnet, daß als Halogenierungsmittel Chlor, Brom oder Sulfurylchlorid eingesetzt wird, bei einer Temperatur von – 20 bis +20°C halogeniert wird und zur Dehydrohalogenierung eine organische Base gegebenenfalls in einem inerten Lösungsmittel eingesetzt wird.

6. Verfahren gemäß Anspruch 4, dadurch gekennzeichnet, daß als Halogenierungsmittel Sulfurylchlorid oder Chlor verwendet wird und die Dehydrohalogenierung durch Erhitzen unter vermindertem Druck ohne Basenzusatz durchgeführt wird.

7. Verfahren gemäß Anspruch 4, dadurch gekennzeichnet, daß pro Mol β-Fluoracylvinylalkylether der Formel (II) 1,0 bis 1,1 Mol Halogenierungsmittel eingesetzt wird.

8. Verfahren gemäß Anspruch 5, dadurch gekennzeichnet, daß pro Mol Verbindung der Formel (III) 1,0 bis 1,5 Mol organische Base eingesetzt wird.

9. Verfahren gemäß Anspruch 5, dadurch gekennzeichnet, daß als organische Base ein tertiäres Amin oder ein Amidin verwendet wird.

10. Verfahren gemäß Anspruch 5, dadurch gekennzeichnet, daß als organische Base Triethylamin eingesetzt wird.

**Claims**

1. β-Fluoroacyl-β-halogenovinyl alkyl ethers of the general formula (I)

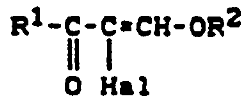
(I)

in which

$R^1$ represents a fluorinated alkyl radical having 1 to 9 carbon atoms,
Hal represents a halogen atom and
$R^2$ represents an alkyl radical having 1 to 7 carbon atoms.

2. β-Fluoroacyl-β-halogenovinyl alkyl ethers according to formula (I) in Claim 1), in which

$R^1$ represents a fluorinated alkyl radical having 1 to 3 carbon atoms
Hal represents chlorine or bromine and
$R^2$ represents an alkyl radical having 1 to 4 carbon atoms.

3. β-Fluoroacyl-β-halogenovinyl ethers according to Claim 2), characterised in that

$R^1$ represents a perfluorinated alkyl radical having 1 to 3 carbon atoms.

4. Process for the preparation of β-fluoroacyl-β-halogenovinyl alkyl ethers of the general formula (I)

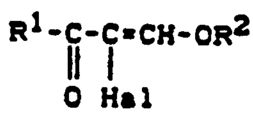
(I)

in which

$R^1$ represents a fluorinated alkyl radical having 1 to 9 carbon atoms,
Hal represents a halogen atom and
$R^2$ represents an alkyl radical having 1 to 7 carbon atoms,

characterised in that β-fluoroacylvinyl alkyl ethers of the formula (II)

$$R^1\text{-CO-CH=CH-OR}^2 \quad [II]$$

where
$R^1$ and $R^2$ have the abovementioned meaning, are reacted with a halogenating agent at temperatures from

−70°C to +80°C, if appropriate in the presence of a diluent, and, if appropriate, the resulting halogenation product of the general formula (III)

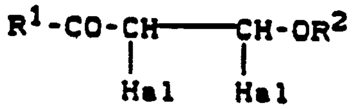

(III)

is isolated, and dehydrohalogenation is carried out at a temperature from −20°C to +100°C,

5. Process according to Claim 4, characterised in that chlorine, bromine or sulphuryl chloride is employed as the halogenating agent, halogenation is carried out at a temperature from −20 to +20°C, and an organic base is employed for dehydrohalogenation, if appropriate in an inert solvent.

6. Process according to Claim 4, characterised in that sulphuryl chloride or chlorine is used as the halogenating agent, and dehydrohalogenation is carried out by heating under reduced pressure without the addition of a base.

7. Process according to Claim 4, characterised in that 1.0 to 1.1 moles of halogenating agent are employed per mole of β-fluoroacylvinyl alkyl ether of the formula (II).

8. Process according to Claim 5, characterised in that 1.0 to 1.5 moles of organic base are employed per mole of compound of the formula (III).

9. Process according to Claim 5, characterised in that a tertiary amine or an amidine is used as the organic base.

10. Process according to Claim 5, characterised in that triethylamine is employed as the organic base.

## Revendications

1. Ethers β-fluoracyl-β-halogénovinylalkyliques de formule générale I :

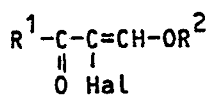

(I)

dans laquelle

$R^1$ représente un groupe alkyle fluoré en $C_1$-$C_9$,
Hal représente un atome d'halogène et
$R^2$ représente un groupe alkyle en $C_1$-$C_7$.

2. Ethers β-fluoracyl-β-halogénovinylalkyliques de formule I de la revendication 1, dans laquelle

$R^1$ représente un groupe alkyle fluoré en $C_1$-$C_3$,
Hal représente le chlore ou le brome et
$R^2$ représente un groupe alkyle en $C_1$-$C_4$.

3. Ethers β-fluoracyl-β-halogénovinyliques selon la revendication 2, caractérisés en ce que

R1 représente un groupe alkyle perfluoré en $C_1$-$C_3$.

4. Procédé de préparation des éthers β-fluoracyl-β-halogénovinylalkyliques de formule générale I :

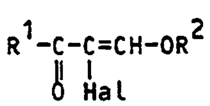

(I)

dans laquelle

$R^1$ représente un groupe alkyle fluoré en $C_1$-$C_9$,
Hal représente un atome d'halogène et
$R^2$ représente un groupe alkyle en $C_1$-$C_7$,

caractérisé en ce que l'on fait réagir des éthers β-fluoracylvinylalkyliques de formule II :

$$R^1\text{-CO-CH=CH-OR}^2 \quad [II]$$

dans laquelle

$R^1$ et $R^2$ ont les significations indiquées ci-dessus, avec un agent halogénant à des températures allant de −70 à +80°C, éventuellement en présence d'un diluant, ce qui donne un produit d'halogénation répondant à la formule générale III :

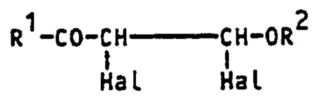

(III)

qu'on isole éventuellement et qu'on soumet à déshydrohalogénation à une température allant de −20 à +100°C.

5. Procédé selon la revendication 4, caractérisé en ce que l'on utilise en tant qu'agent halogénant le chlore, le brome ou le chlorure de sulfuryle, on halogène a une température de −20 à +20°C et on utilise pour la déshydrohalogénation une base organique éventuellement dans un solvant inerte.

6. Procédé selon la revendication 4, caractérisé en ce que l'on utilise en tant qu'agent halogénant le chlorure de sulfuryle ou le chlore et on procède à la déshydrohalogénation par chauffage sous vide sans addition d'une base.

7. Procédé selon la revendication 4, caractérisé en ce que l'on utilise de 1,0 à 1,1 mol d'agent halogénant par mole de l'éther β-fluoracylvinylalkylique de formule II.

8. Procédé selon la revendication 5, caractérisé en ce que l'on utilise de 1,0 a 1,5 mol de base organique par mole du composé de formule III.

9. Procédé selon la revendication 5, caractérisé en ce que l'on utilise en tant que base organique une amine tertiaire ou une amidine.

10. Procédé selon la revendication 5, caractérisé en ce que la base organique utilisée est la triéthylamine.